# EUROPEAN PATENT APPLICATION

(11) **EP 1 319 649 A1**
(43) Date of publication of application: **18.06.2003**
(21) Application number: 02258417.1
(22) Date of filing: 05.12.2002
(51) Int. Cl.: C07C 51/50, C07C 57/04, C07C 51/46

(54) **Process for inhibiting polymer formation in an vinyl monomer azeotropic dehydration process**

(30) Priority: 13.12.2001 US 340458 P
(71) Applicant: ROHM AND HAAS COMPANY, Philadelphia, Pennsylvania 19106-2399 (US)
(72) Inventor: Chalfant, David Clark, Levittown, Pennsylvania 19056 (US); Day, James Clarence, English Village, North Wales, Pennsylvania 19454 (US); Grieco, William Joseph, Collegeville, Pennsylvania 19426-4116 (US)
(74) Representative: Kent, Venetia Katherine

(57) **Abstract**

An improved process is provided herein for the purification of polymerizable organic material, e.g., acrylic acid, by azeotropic distillation.

## Description

The present invention is related to a process for purifying polymerizable organic material wherein undesirable polymerization is minimized or eliminated. More particularly, the present invention relates to a process for purifying polymerizable organic material such as acrylic acid ("AA"), water-soluble esters of acrylic acid, methacrylic acid ("MAA"), water-soluble esters of methacrylic acid and other water-soluble vinyl monomers.

Purifying polymerizable organic material is commonly accomplished via distillation in the presence of a polymerization inhibitor. Traditionally, such a purification process involves the addition of polymerization inhibitors in various locations throughout the distillation apparatus. To be effective, the polymerization inhibitor must be uniformly distributed throughout the column so that there is adequate inhibitor in all locations where polymerizable material exists. However, despite advances in tray design, inhibitor insertion techniques, oxygen addition, and heat addition, uniform distribution of inhibitor is difficult to achieve and polymerizable organic material may be inadequately inhibited during distillation. Lack of adequate polymerization inhibitor allows the polymerizable organic material to polymerize into undesirable solids in the column.

European Published Patent Application 1 035 103 A2 discloses a process for the preparation of acrylic acid wherein an aqueous acrylic feed stream is purified by azeotropic distillation with toluene solvent. In a working example, the reference discloses the purification of an aqueous acrylic acid feed composition comprising 67 wt % acrylic acid, 1 wt % β-acryloxy-propionic acid (AOPA), 28 wt % water, 3 wt % acetic acid-and 1 wt % other minor components such as formaldehyde, formic acid, maleic acid and hydroquinone polymerization inhibitor. Also, 4-hydroxy-2,2,6,6-tetramethylpiperidinyloxy free radical polymerization inhibitor was fed as a 0.08 wt % aqueous solution into the aqueous acrylic acid feed and p-benzoquinone vapor phase inhibitor was fed as a 0.24 wt % toluene solution to the top tray of the distillation column. Furthermore, an additional stream of 4-hydroxy-2,2,6,6-tetramethylpiperidinyloxy free radical was fed as a 0.34 wt % aqueous solution to the top tray of the distillation column. After an extended distillation run, it was reported that the column and pot were clean, i.e. no polymerization of monomer detected. However, it has now been determined that while no polymer build-up occurred on the surfaces of the equipment utilized, as reported, in fact, small amounts of finely divided particulate polymer (polyacrylic acid) were formed in the bottoms product.

Unwanted polymer must be physically removed via the strategic insertion of strainers in the column and/or regularly shutting down the column to allow personnel to have access for the manual removal of polymer buildup. These solutions are time consuming, costly, and inefficient. Consequently, a great need exists for a method of more efficiently minimizing or eliminating polymerization in distillation apparatus used to purify polymerizable organic material.

The present invention solves the problems inherent in the prior art by providing an economical method for more effectively minimizing or eliminating polymerization in distillation apparatus used to purify polymerizable organic material. More particularly, the present invention provides an improved method for minimizing or eliminating polymerization in a distillation apparatus used to purify polymerizable organic material. The improved method comprises providing a distillation column; feeding to said distillation column an unpurified aqueous organic stream comprising polymerizable organic material and water; feeding one or more inhibitor streams to said distillation column, an inhibitor stream comprising polymerization inhibitor and a delivery solvent; feeding a water-insoluble distillation solvent to said distillation column to induce azeotropic distillation; and azeotropically distilling said unpurified aqueous organic stream in said distillation column to form a purified product stream; wherein the improvement comprises: at least one of the one or more inhibitor streams comprising a polymerization inhibitor which is soluble in both water and organic materials and a delivery solvent which is insoluble in water.

The method of the present invention is advantageous because it allows an unpurified aqueous polymerizable organic stream to be purified without forming undesirable polymer. Other and further objects, features, and advantages will be apparent from the following description of some embodiments of the invention.

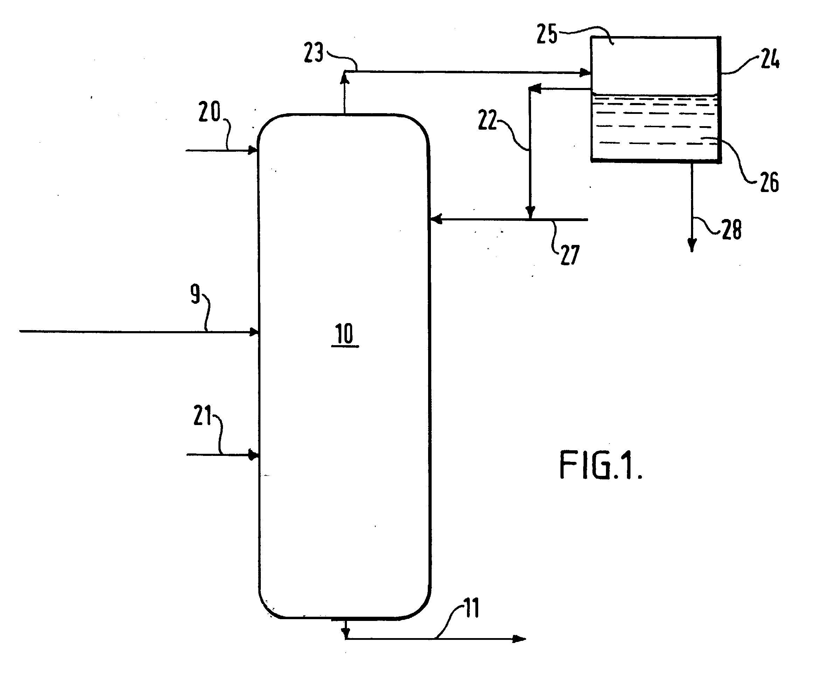

A more complete understanding of the present embodiments and advantages thereof may be acquired by referring to the following description taken in conjunction with accompanying Figure 1.. Figure 1 is a diagram of an embodiment of the present invention depicting a system for minimizing or eliminating polymerization in a distillation apparatus used to purify aqueous polymerizable organic material.

The present invention involves purifying polymerizable organic material including, but not limited to, acrylic acid ("AA"), water-soluble esters of acrylic acid, methacrylic acid ("MAA"), water-soluble esters of methacrylic acid and other water-soluble vinyl monomers. Specifically, this invention relates to methods of purifying polymerizable organic material wherein undesirable polymerization is minimized or eliminated.

The present invention involves the purification of a stream of aqueous polymerizable organic material, stream **9.** In one embodiment of the present invention in which AA is the aqueous polymerizable organic material being purified, stream **9** comprises from 20 to 95, preferably 35 to 90, more preferably 50 to 80 percent by weight AA; from 80 to 5, preferably from 65 to 10, more preferably from 50 to 20 percent by weight water; and up to 8, preferably up to 6, more preferably up to 5 percent by weight acetic acid.

Aqueous polymerizable organic material in stream **9** is fed to a distillation column **10** in which the organic polymerizable material is separated from the water. Distillation column **10** may be any suitable distillation column known in the art. For instance, a sieve tray column, a dual flow tray column, or a packed column may be used. In distillation column **10**, aqueous polymerizable organic stream **9** is subjected to azeotropic distillation in the presence of at least one distillation solvent to form purified product stream **11**, which is substantially free of water. In one embodiment of the present invention wherein the material being purified is AA, purified product stream **11** generally has less than 1,000, preferably less than 800 and more preferably less than 500, ppm water.

The distillation solvent or solvents may include any solvent suitable for the azeotropic distillation of the polymerizable organic material being distilled. The distillation solvent is introduced to distillation column **10** via distillation solvent feed line **27**. In one embodiment of the present invention wherein the polymerizable organic material is AA, the distillation solvent is substantially water insoluble, generally having a solubility in water at room temperature of 0.5 weight percent or less, preferably 0.2 weight percent or less. Suitable examples of such water insoluble solvents include but are not limited to: heptane; cycloheptane; cycloheptene; cycloheptatriene; methylcyclohexane; ethylcyclopentane; 1,2-dimethylcyclohexane; ethylcyclohexane; toluene; ethylbenzene; ortho-xylene; meta-xylene; para-xylene; trichloroethylene; trichloropropene; 2,3-dichlorobutane; 1-chloropentane; 1-chlorohexane; chlorobenzene; ethyl acetate; butyl acetate; dibutyl ether; hexane; heptane; ethyl methacrylate; diethyl ketone; methyl propyl ketone; and methyl tert-butyl ketone. In some embodiments of the present invention, the distillation solvent may be a mixed solvent comprising one or more of the above-mentioned compounds.

Polymerization inhibitors may be introduced to the column in one or more inhibitor streams . Such inhibitor streams may enter distillation column **10** above or below aqueous polymerizable organic stream **9**, or both above and below aqueous polymerizable organic stream **9**. In one embodiment of the present invention, inhibitor is introduced to distillation column **10** through inhibitor streams **20** and **21**. However, at least one of the inhibitor streams comprises a polymerization inhibitor which is soluble in both water and organic materials and a delivery solvent which is insoluble in water. Polymerization inhibitors that are soluble in both aqueous and organic materials include but are not limited to: hydroquinone ("HQ"); 4-methoxyphenol; 4-ethoxyphenol; 1,2-dihydroxybenzene; catechol monobutyl ether; pyrogallol; 4-aminophenol; 2-mercaptophenol; 4-mercaptophenol; 4-hydroxy-2,2,6,6-tetramethyl piperidinyloxy free radical; 4-oxo-2,2,6,6-tetramethyl piperidinyloxy free radical; 4-amino-2,2,6,6-tetramethyl piperidinyloxy free radical; or mixtures of two or more thereof; or mixtures of one or more of the above with oxygen. Use of oxygen is preferred when phenolic inhibitors, such as HQ are used, as the oxygen enhances the effectiveness of the inhibitor. Oxygen may be added in one or more locations throughout distillation column **10.** Operating temperatures and pressures impact the flammability limits and oxygen solubility within the purification system, and these properties must be taken into account when determining the appropriate oxygen concentration to be used for the oxygen-containing gas. Considerations of such factors are within the ability of one of ordinary skill in the art, and either pure oxygen or atmospheric air may be commonly employed. High oxygen concentrations within the monomer-containing solution itself should be avoided. When oxygen concentrations are large relative to inhibitor concentrations, oxygen can actually increase the rate of polymerization by promoting the formation of peroxides and, ultimately, monomer radicals. For this reason, it is not recommended that oxygen be added when no inhibitor is present. The optimal oxygen to inhibitor ratio will vary with respect to the inhibitor used.

Polymerization inhibitor is generally purchased as a bulk powder and must be mixed with a delivery solvent before introduction into distillation column **10**. Conventionally, the delivery solvents used were aqueous, preferably water. Japanese patent application EP 976702 discloses the use of water as an effective delivery solvent for N-oxyl compound inhibitor in distillation purification of acrylic acid. Likewise, German patent application DE 19954582 cites water as one of a number of inert solvents for use of N-oxyl compound inhibitor in stabilization of acrylic acid. While polymer inhibition results are expected to be insensitive to the particular choice of delivery solvent, we have discovered that the use of an inhibitor soluble in both aqueous and organic materials together with a water insoluble delivery solvent in at least one of the inhibitor feeds yields substantially less unwanted polymerization. For instance, using a toluene delivery solvent in conjunction with 4-hydroxy-2,2,6,6-tetramethyl piperidinyloxy free radical inhibitor in one inhibitor feed, while utilizing water as the delivery solvent in another inhibitor feed for the 4-hydroxy-2,2,6,6-tetramethyl piperidinyloxy free radical inhibitor, results in no detectable polymerization; whereas the use of water as the delivery solvent, in both feeds, with 4-hydroxy-2,2,6,6-tetramethyl piperidinyloxy free radical inhibitor results in small, but detectable levels of undesirable polymerization.

According to the present invention, delivery solvents which are substantially water insoluble, generally have a solubility in water at room temperature of 0.5 weight percent or less, preferably 0.2 weight percent or less. It may also be desirable to use the same inhibitor delivery solvent as the distillation solvent. Suitable examples of such water insoluble delivery solvents include but are not limited to: heptane; cycloheptane; cycloheptene; cycloheptatriene; methylcyclohexane; ethylcyclopentane; 1,2-dimethylcyclohexane; ethylcyclohexane; toluene; ethylbenzene; ortho-xylene; meta-xylene; para-xylene; trichloroethylene; trichloropropene; 2,3-dichlorobutane; 1-chloropentane; 1-chlorohexane; 1-chlorobenzene; ethyl acetate; butyl acetate; dibutyl ether; hexane; heptane; ethyl methacrylate; diethyl ketone; methyl propyl ketone; and methyl tert-butyl ketone. In some embodiments of the present invention, the solvent may be a mixed solvent comprising more than one of the above-mentioned compounds. Toluene is a preferred delivery solvent.

Thus, in an embodiment of the present invention, aqueous polymerizable organic material in stream **9** is fed into distillation column **10** along with a distillation solvent introduced to the column via distillation solvent feed line **27**. Inhibitor (soluble in both aqueous and organic material) along with a water-insoluble delivery solvent is introduced to the column via inhibitor feed lines **20** and **21**. Purified product stream **11** exits the bottom of the column and is substantially water-free. Overhead stream **23** exits the top of the column and enters overhead tank **24**. Once inside overhead tank **24**, the material is allowed to separate into an aqueous phase **26** and an organic phase **25**. Such phase separation may be accomplished by various means known in the art. Organic phase **25** is predominantly comprised of distillation solvent and delivery solvent, while aqueous phase **26** is predominantly comprised of water and light impurities. As organic phase **25** is predominately distillation and delivery solvent, it may be recycled into column **10** through organic recycle line **22** to supplement the fresh distillation and delivery solvents entering column **10**. Aqueous impurities are removed from distillation column **10** through waste water line **28.**

### Comparative Example 1: Azeotropic distillation with a 4-HT-water inhibitor feed.

An azeotropic toluene distillation column run was conducted at operating conditions using a 1 inch diameter, 30-tray Oldershaw column mounted on a bottoms reboiler pot sparged with air at a rate of 68 cc/min. The feed tray was at tray 15, and the control tray was at tray 18, both from the bottom. The distillation was operated at the following conditions:
215 mm Hg top pressure
322 g/hr aqueous AA feed rate
649.5 g/hr toluene reflux rate
77°C control tray temperature
An aqueous acrylic acid feed composition was fed to the distillation column at tray 15 and toluene reflux was fed to the top tray at the rate indicated. The aqueous acrylic acid feed composition contained 68.9 wt% acrylic acid, 0.7 wt% beta-acryloxypropionic acid (AOPA), 26.4 wt% water, and 2.9 wt% acetic acid, and 1.1% other minor components such as formaldehyde, formic acid, maleic acid, and hydroquinone polymerization inhibitor. A 0.3 wt% aqueous solution of 4-hydroxy-2,2,6,6-tetramethyl piperidinyloxy free radical polymerization inhibitor, available from Aldrich Chemical Co. of Milwaukee, Wisconsin, was fed into the aqueous acrylic acid at a rate of 3 g/hr. An additional stream of 0.6 wt% aqueous solution of 4-hydroxy-2,2,6,6-tetramethyl piperidinyloxy free radical was fed to the top tray at a rate of 6 g/hr. The inhibitor feeds resulted in inhibitor levels in the bottoms of 200 ppm 4-hydroxy-2,2,6,6-tetramethyl piperidinyloxy free radical and 323 ppm hydroquinone. Bottoms product was collected at a rate of 224 g/hr and contained 93.4 wt% acrylic acid, 6.0 wt% beta-acryloxypropionic acid, 652 ppm acetic acid, and 5 ppm toluene. Aqueous distillate was collected at a rate of 107 g/hr and contained 89.5% water, 1.9 wt% acrylic acid, 8.6 wt% acetic acid, and 262 ppm toluene.

At the end of the 8 hour run, the column was clean, but the bottoms product contained minor amounts of finely divided white solids which were collected and shown by IR analysis to be polyacrylic acid (undesirable polymer)

### Example 2: Azeotropic distillation with a 4-HT-toluene inhibitor feed.

An azeotropic toluene distillation column run was conducted at operating conditions using a 1 inch diameter, 30-tray Oldershaw column mounted on a bottoms reboiler pot sparged with air at a rate of 68 cc/min. The feed tray was at tray 15, and the control tray was at tray 18, both from the bottom. The distillation was operated at the following conditions:
215 mm Hg top pressure
322 g/hr aqueous AA feed rate
610.1 g/hr toluene reflux rate
77°C control tray temperature
An aqueous acrylic acid feed composition was fed to the distillation column at tray 15 and toluene reflux was fed to the top tray at the rate indicated. The aqueous acrylic acid feed composition contained 68.9 wt% acrylic acid, 0.7 wt% beta-acryloxypropionic acid (AOPA), 26.4 wt% water, and 2.9 wt% acetic acid, and 1.1% other minor components such as formaldehyde, formic acid, maleic acid, and hydroquinone polymerization inhibitor. A 0.3 wt% aqueous solution of 4-hydroxy-2,2,6,6-tetramethyl piperidinyloxy free radical polymerization inhibitor, available from Aldrich Chemical Co. of Milwaukee, Wisconsin, was fed into the aqueous acrylic acid at a rate of 3 g/hr. An additional stream of 4-hydroxy-2,2,6,6-tetramethyl piperidinyloxy free radical, 0.6 wt% in toluene, was fed to the top tray at a rate of 6 g/hr. The inhibitor feeds resulted in inhibitor levels in the bottoms of 200 ppm 4-hydroxy-2,2,6,6-tetramethyl piperidinyloxy free radical and 323 ppm hydroquinone. Bottoms product was collected at a rate of 224 g/hr and contained 93.3 wt% acrylic acid, 6.1 wt% beta-acryloxypropionic acid, 757 ppm acetic acid, and 42 ppm toluene. Aqueous distillate was collected at a rate of 101 g/hr and contained 90.0 wt% water, 1.7 wt% acrylic acid, 8.3 wt% acetic acid, and 353 ppm toluene.

At the end of the 8-hour run the column was clean and bottoms product was also clear and free of any suspended solids.

The present invention, therefore, is well adapted to carry out the objects and attain both the ends and the advantages mentioned, as well as other benefits inherent therein. The present invention allows for the purification of an aqueous polymerizable organic material such as AA, MAA, or other water-soluble vinyl monomers while minimizing or eliminating the formation of undesirable polymer. While the present invention has been depicted, described, and is defined by reference to particular embodiments of the invention, such references do not imply a limitation on the invention, and no such limitation is to be inferred. The invention is capable of considerable modification, alteration, and substitution of equivalents in form and/or function, as will occur to those of ordinary skill in the pertinent arts. The depicted and described embodiments of the invention are exemplary only and are not exhaustive of the scope of the invention. Consequently, the invention is intended to be limited only by the spirit and scope of the appended claims, giving full cognizance to equivalents in all respects.

## Claims

1. An improved process for azeotropically purifying aqueous polymerizable organic material comprising
providing a distillation column;
feeding to said distillation column an unpurified aqueous organic stream comprising polymerizable organic material and water;
feeding one or more inhibitor streams to said distillation column, each inhibitor stream comprising polymerization inhibitor and a delivery solvent;
feeding a water-insoluble distillation solvent to said distillation column to induce azeotropic distillation; and
azeotropically distilling said unpurified aqueous organic stream in said distillation column to form a purified product stream;
wherein the improvement comprises:
at least one of said one or more inhibitor streams comprising a polymerization inhibitor which is soluble in both water and organic materials and a delivery solvent which is insoluble in water.

2. The process for azeotropically purifying aqueous polymerizable organic material of claim 1 wherein the polymerization inhibitor which is soluble in both water and organic materials is selected from the group consisting of hydroquinone; 4-methoxyphenol; 4-ethoxyphenol; 1,2-dihydroxybenzene; catechol monobutyl ether; pyrogallbl; 4-aminophenol; 2-mercaptophenol; 4-mercaptophenol; 4-hydroxy-2,2,6,6-tetramethyl piperidinyloxy free radical; 4-oxo-2,2,6,6-tetramethyl piperidinyloxy free radical; 4-amino-2,2,6,6-tetramethyl piperidinyloxy free radical; and mixtures of two or more thereof.

3. The process for azeotropically purifying aqueous polymerizable organic material of claim 2 wherein the polymerization inhibitor which is soluble in both water and organic materials is 4-hydroxy-2,2,6,6-tetramethyl piperidinyloxy free radical.

4. The process for azeotropically purifying aqueous polymerizable organic material of claim 1 wherein the delivery solvent which is insoluble in water is selected from the group consisting of heptane; cycloheptane; cycloheptene; cycloheptatriene; methylcyclohexane; ethylcyclopentane; 1,2-dimethylcyclohexane; ethylcyclohexane; toluene; ethylbenzene; ortho-xylene; meta-xylene; para-xylene; trichloroethylene; trichloropropene; 2,3-dichlorobutane; 1-chloropentane; 1-chlorohexane; chlorobenzene; ethyl acetate; butylacetate; dibutyl ether; hexane; heptane; ethyl methacrylate; diethyl ketone; methyl propyl ketone; methyl tert-butyl ketone; and mixtures of two or more thereof.

5. The process for azeotropically purifying aqueous polymerizable organic material of claim 4 wherein the delivery solvent which is insoluble in water is toluene.

6. The process for azeotropically purifying aqueous polymerizable organic material of claim 1 wherein the distillation apparatus is selected from the group consisting of a packed distillation column, a distillation column with dual flow trays, and a distillation column with sieve trays.

7. The process for azeotropically purifying aqueous polymerizable organic material of claim 1 wherein said polymerizable organic material is a vinyl monomer.

8. The process for azeotropically purifying aqueous polymerizable organic material of claim 7 wherein said vinyl monomer is acrylic acid.
